Europäisches Patentamt

**European Patent Office**

**Office européen des brevets**

(11) Publication number: **0 005 038**
**A2**

# EUROPEAN PATENT APPLICATION

(21) Application number: **79300622.2**

(22) Date of filing: **12.04.79**

(51) Int. Cl.²: **A 61 B 5/10**
**A 43 D 1/02**

(30) Priority: **24.04.78 GB 1609378**

(43) Date of publication of application:
**31.10.79 Bulletin 79/22**

(84) Designated Contracting States:
**BE CH DE FR IT LU NL SE**

(71) Applicant: **MALTHOUSE RANGER LIMITED**
**2 West Hill Road**
**Bournemouth Dorset(GB)**

(72) Inventor: **Snook, Laurence Arthur**
**2 St. James Lane**
**Winchester Hampshire(GB)**

(74) Representative: **Topps, Ronald et al,**
**D. Young & Co 9 & 10 Staple Inn**
**London WC1V 7RD(GB)**

(54) **Automatic foot gauge.**

(57) An automatic gauge for measuring a person's foot and for giving an indication of a suitable size of footwear for the measured foot, the gauge comprising a first pair of members (15 and 38) of which the member (38) is movable towards and away from the member (15), a second pair of members (13 and 28) of which the member (28) is movable towards and away from the member (13), the foot (T) to be measured being placed between the members (15) and (38) and between the members (13) and (28), said gauge including electrical means (32, 33) and (34, 35) and circuit means (20) for processng the electrical output signals from the electrical means (32, 33 and 34, 35) and means (45 and 49) for displaying a size of footwear related to the spacing between the members of the pairs of members (15, 38 and 13, 28).

FIG. 1.

EP 0 005 038 A2

Croydon Printing Company Ltd.

0005038

1

# AUTOMATIC FOOT GAUGE

This invention relates to a gauge for measuring a person's foot and for giving an indication of a suitable size of footwear for the measured foot.

Manually operable foot gauges are known in which the foot is placed between a heel stop and a slide member which give an indication of foot length, a flexible tape being passed over the foot to give an indication of foot width. Such gauges depend for accuracy on the skill of the operator and measurements are taken when the person whose foot is being measured is sitting. Consequently as there is no weight placed on the foot the measurements are taken with the foot in a relaxed condition and not with the foot supporting body weight.

There is a need for a foot gauge which is automatic in use and not subject to the skill of an operator and which can be used to measure a foot whilst a person whose foot is being measured is standing.

According to the present invention there is provided a gauge for measuring a person's foot and for giving an indication of a suitable size of footwear for the measured foot, comprising a first pair of members between which the heel and the toe ends of a foot can be placed, one of said first members being movable towards and away from the other member, a second pair of members between which the sides of a foot can be placed, one of said second members being movable towards and away from the other second member, electrical means responsive to the spacing between the members of said pairs of members, circuit means and means for displaying a size of footwear related to the spacing between the members of said pairs of members.

Preferably the movable member of said first pair of members is provided on a slide member movable along a slide provided on a base member and the movable member of said second pair of members is provided on a slide bar movable on the slide member in a direction at right angles to the direction of movement of the slide member.

The other member of the first pair of members is preferably formed by a side wall of a recess provided in said base member and in which the foot is placed and the other member of the second pair of members is formed by a pivoted plate member connected to an on/off switch for the electrical means.

In a preferred arrangement the electrical means responsive to the spacing between said first pair of members comprises a contact brush carried by the movable member of said first pair of members, a row of electrical contacts along which the contact brush can be moved by the movable member of said first pair of members, an encoder electrically connected to all electrical contacts and a display means electrically connected to the encoder.

Preferably the electrical means responsive to the spacing between said second pair of members comprises a contact brush carried by the movable member of said second pair of members, an encoder electrically connected to all contact strips and a display means electrically connected to the encoder.

An embodiment of the present invention will now be described, by way of an example, with reference to the accompanying drawings, in which:-

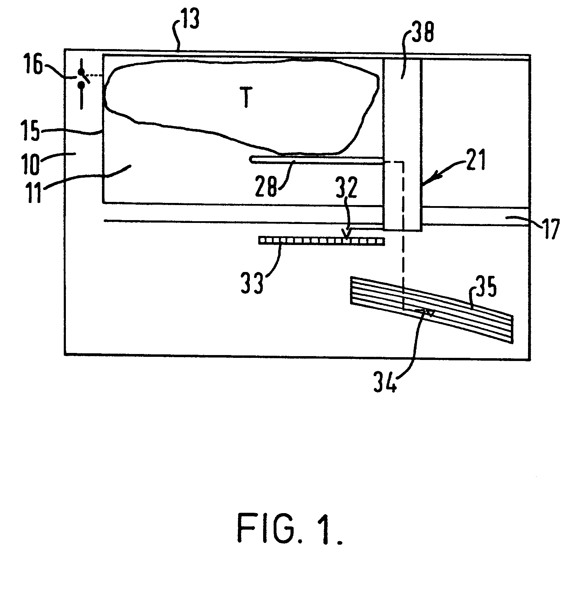

Figure 1 is a diagrammatic representation of the gauge according to the present invention,

Figure 2 is a diagram of the electrical circuit for measuring the length of the foot,

Figure 3 is a diagram of the electrical circuit for measuring the width of the foot,

Figure 4 is an exploded perspective view of the component parts of the gauge, and

Figure 5 is an exploded perspective view of the measuring slide assembly in greater detail.

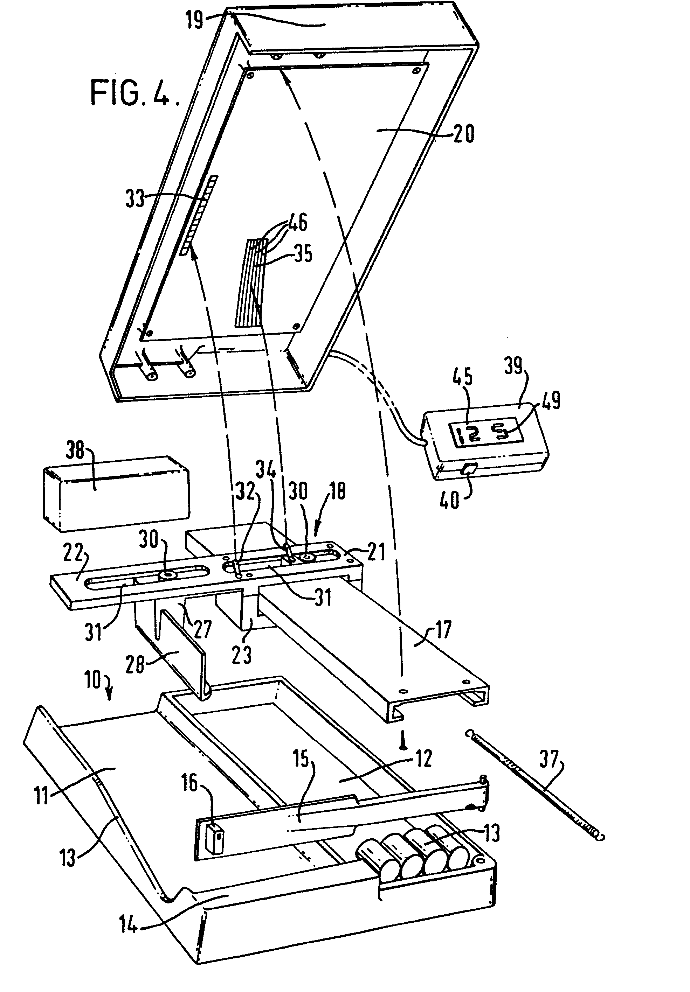

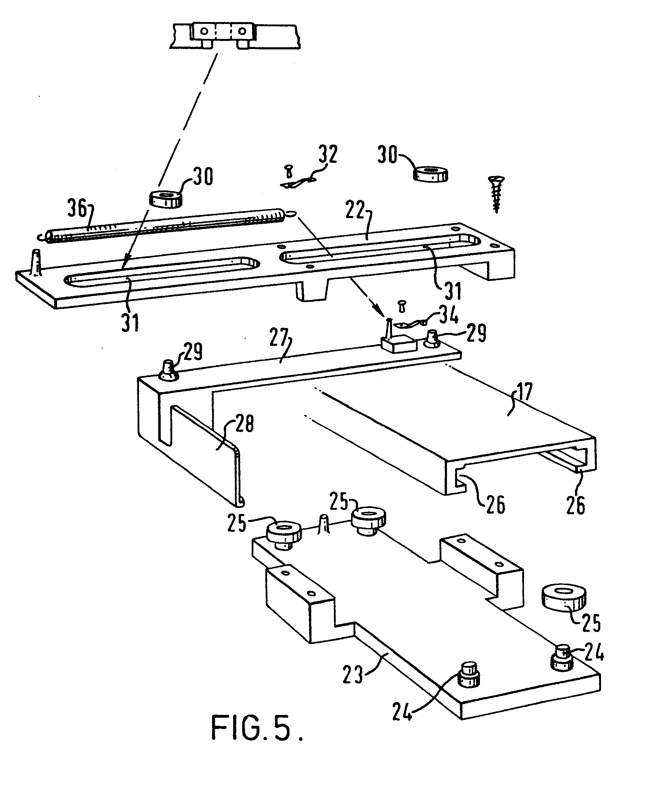

Referring first of all to Figures 4 and 5 it will be seen that the gauge comprises a base member 10 in which is provided a recess 11 and a recess 12. The recess 11 is provided with a side wall 13 and an end wall 14. Extending in front of the end wall 14 is a pivoted member 15 which carries a microswitch 16 which as described hereinafter operates as an on/off switch for the electrical circuit of the gauge. Located in the recess 12 and fixed to the base member 10 is a channel section slide 17 along which can move a measuring bar assembly 18 which is shown in greater detail in Figure 5. Also housed in the recess 12 are batteries 13 which serve as the source of electrical energy for the gauge. The batteries 13 may be of the re-chargable type.

Instead of the electrical energy being obtained from batteries it is of course possible to design the gauge so that it can be supplied with electrical current from the mains, preferably through a transformer.

The base member 10 is preferably formed of a plastics material which may be fibre reinforced, such as by glass fibres. Secured to the base member 10 is a cover member 19 also preferably formed of a plastics material which may be fibre reinforced. The cover member 19 carries a printed circuit board 20 which in turn carries encoding circuitry and encoders to be described hereafter.

The measuring bar assembly 18 consists of a carriage 21 formed by an upper moulding 22 and a lower moulding 23 which is provided with upstanding spigots 24 on which are press fitted ball races 25 which engage in the channels 26 of the slide 17. The ball races 25 constrain the carriage 21 to move only longitudinally of the slide 17. Located between the mouldings 22 and 23 and extending above the slide 17 is a bar 27 provided at one end with a portion 28 which extends at a right angle to the carriage 21 and is parallel with the side wall 13. The bar 27 is provided with spigots 29 on which are press fitted ball races 30 which are received in elongate slots 31 provided in the upper moulding 22 and constrain the bar 27 to move only longitudinally of the upper moulding 22.

The upper moulding 22 carries an electrical contact brush 32 which makes contact with a contact area 33 provided on the underside of the printed circuit board 20 and the bar 27 carries an electrical contact brush 34 which makes contact with a contact area 35 provided on the underside of the printed circuit board 20.

A light spring 36 extends between the upper moulding 22 and the bar 27 and a light spring 37 extends between the carriage 21 and the base member 10. The spring 36 urges the bar 27 towards the side wall 13 and the spring 37 urges the carriage 21 towards the end wall 14. Mounted on the upper moulding 22 is a cover 38 having a smooth vertical surface which in use is pressed against the toes of the foot being measured.

The gauge also includes a hand held display unit 39 which includes a switch 40 so that readings are only displayed after the foot is properly settled in position.

In use of the gauge the foot T to be measured is placed in the recess 11 with the heel against the pivoted member 15 which is pivoted towards the end wall 14 to operate the microswitch 16 which energizes the electrical circuits to be described hereafter. The toe end of the foot is contacted by the cover 38 and the carriage 21 is moved along the slide 17 against the pressure of the spring 37. One side of the foot is placed against the side wall 13 and the other side of the foot is contacted by the portion 28 of the bar 27 which is moved along the upper moulding 22 against the pressure of the spring 36.

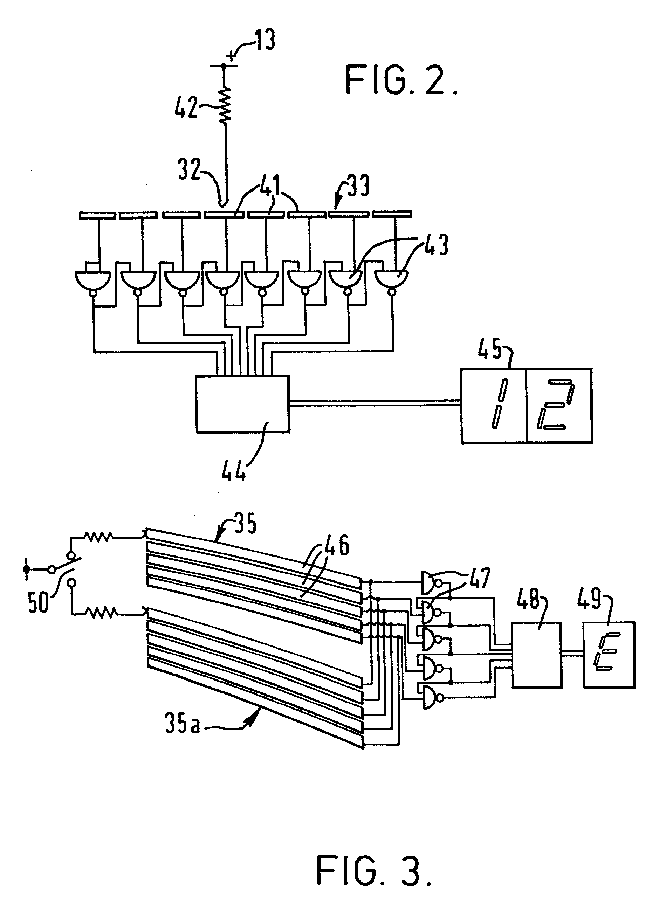

Referring now in greater detail to Figure 2 the contact area 33 of the printed circuit board 20 comprises a plurality of contact pads 41. The contact brush 32 is connected via a current limiting resistor 42 to the common voltage supply source 13 and therefore supplies a "Logic 1" signal into whichever of the contact pads 41 it rests on. In order to avoid ambiguous indication when the brush bridges the gap between two pads 41 the output from each pad 41 is combined in a "NAND" gate 43 with the inverted output of the pad corresponding to the next larger reading. Thus when the contact brush 32 makes simultaneous contact with two adjacent pads 41 only that pad corresponding to the larger shoe size gives an output. The selected output is fed via an encoder 44 to a read-out 45 on the display unit 39, on which the shoe size corresponding to the length of the measured foot T is displayed.

The contact area 35 consists of strips 46 which lie at an angle relative to the direction of movement of the slide 21. The contact brush 34 moves across the strips 46 and therefore there is given the relationship of width to length for the various shoe width fittings. The output from each contact strip 46 is processed through "NAND" gates 47 and an encoder 48 to display the shoe width fitting on a further read-out device 49 on the display unit 39.

As shown in Figure 3, where two or more size ranges are required to be measured on the same gauge but do not share a common width distribution, such as for gents, ladies and children, two or more width contact areas 35, 35a can be provided on different parts of the printed circuit board 20 and the appropriate brush contact 34, 34a etc. selected by means of a switch 50.

It will be appreciated that the display unit 39 can be mounted on the cover instead of being hand held.

6

## CLAIMS

1.     A gauge for measuring a person's foot and for giving an indication of a suitable size of footwear for the measured foot, characterised in that it comprises a first pair of members (15 and 38) between which the heel and toe ends of a foot (T) can be placed, one (38) of said first members (15 and 38) being movable towards and away from the other member (15), a second pair of members (13 and 28) between which the sides of a foot (T) can be placed, one (28) of said second members (13 and 28) being movable towards and away from the other second member (13), electrical means (32, 33 and 34, 35) responsive to the spacing between the members (15 and 38, 13 and 28) of said pairs of members, circuit means (20) for processing the electrical output signals from said electrical means (32, 33 and 34, 35) and means (45, 49) for displaying a size of footwear related to the spacing between the members of said pairs of members (15, 38 and 13, 28).

2.     A gauge as claimed in claim 1, in which the movable member (38) of said first pair of members (15 and 38) is provided on a slide member (21) movable along a slide (17) provided on a base member (10) and the movable member (28) of said second pair of members (13 and 28) is provided on a slide bar (27) movable on the slide member (21) in a direction at right angles to the direction of movement of the slide member (21).

3.     A gauge as claimed in claim 2, in which the other member (13) of the second pair of members (13 and 28) is formed by a side wall of a recess (11) provided in said base member (10) and in which the foot (T) is placed and the other member (15) of the first pair of members (15 and 28) if formed by a pivoted plate member (15) connected to an on/off switch (16) for the electrical means (32, 33 and 34, 35).

4.    A gauge as claimed in any preceding claim, in which the circuit means (20) is a printed circuit provided on a board.

5.    A gauge as claimed in claim 4, in which the printed circuit board (20) is provided on a cover member (19).

6.    A gauge as claimed in any preceding claim, in which the electrical means (32, 33) responsive to the spacing between said first pair of members (15 and 38) comprises a contact brush (32) carried by the movable member (38) of said first pair of members (15 and 38), a row of electrical contacts (33) along which the contact brush (32) can be moved by the movable member (38) of said first pair of members (15 and 38), an encoder (44) electrically connected to all electrical contacts (33) and a display means (45) electrically connected to the encoder (44).

7.    A gauge as claimed in any preceding claim, in which the electrical means (34, 35) responsive to the spacing between said second pair of members (13 and 28) comprises a contact brush (34) carried by the movable member (28) of said second pair of members (13 and 28), a plurality of parallel contact strips (35) across which the brush (34) can be moved by the movable member (28) of said second pair of members (13 and 28), an encoder (48) electrically connected to all contact strips (35) and a display means (49) electrically connected to the encoder (48).

8.    A gauge as claimed in claim 7 cmprising two or more brushes (34) carried by the movable member (28) of said second pair of members (13 and 28) and a plurality of parallel contact strips (35, 35a) associated with each brush, all of the contact strips (35, 35a) being connected to the encoder (48) and switch means (50) being provided for selecting which of said brushes (34) are supplied with electrical current.

9.    A gauge as claimed in any preceding claim, in which batteries (13) are provided for supplying electrical power.

10.     A gauge as claimed in any preceding claim, in which the movable members (38 and 28) are each resiliently urged towards the other member (15 and 13) of the pair.

11.     A gauge as claimed in any one of claims 6 to 8, in which each contact (33) is connected to the adjacent contact by a NAND gate (43) whose output is connected to the encoder (44).

12.     A gauge as claimed in claim 2 and any claim dependent thereon, in which the slide member (21) is provided with ball races (25) in contact with the slide (17) and the slide bar (27) is provided with ball races (30) movable in elongate slots (31) provided in the slide member (21).

FIG. 1.

FIG. 2.

FIG. 3.

FIG. 4.

0005038

4/4

FIG.5.